# DEMANDE DE BREVET EUROPEEN

(11) **EP 1 114 868 A1**
(43) Date de publication de la demande: **11.07.2001**
(21) Numéro de dépôt: 00403297.5
(22) Date de dépôt: 24.11.2000
(51) Int. Cl.: C12P 19/02, C12P 7/58, C12N 9/04

(54) **Procédé de transformation de matières saccharidiques, comprenant une étape d'oxydation enzymatique en présence de ruthénium ou palladium**

(30) Priorité: 07.12.1999 FR 9915434
(71) Demandeur: Roquette Frères, 62136 Lestrem (FR)
(72) Inventeur: Fouache, Catherine, 62113 Sailly La Bourse (FR); Tamion, Rodolphe, 62157 Allouagne (FR); Fleche, Guy, 59190 Hazebrouck (FR); Moine, Didier, 59660 Merville (FR); Fuertes, Patrick, 59130 Lambersart (FR)
(74) Mandataire: Boulinguiez, Didier

(57) **Abrégé**

Procédé de transformation d'une matière organique comprenant une étape d'oxydation au cours de laquelle une matière organique est soumise à l'action oxydante d'un moyen enzymatique apte à générer du peroxyde d'hydrogène, ce procédé étant caractérisé par le fait que ladite étape d'oxydation est menée, en tout ou partie, en présence d'un métal choisi parmi le ruthénium, le palladium et leurs mélanges.

## Description

La présente invention a pour objet un nouveau procédé de transformation de matières organiques, en particulier de matières saccharidiques, comprenant une étape d'oxydation menée dans des conditions particulières, à savoir associant, au moins à un moment donné, un moyen d'oxydation enzymatique apte à générer du peroxyde d'hydrogène et au moins un métal particulier, en l'occurrence du ruthénium et/ou du palladium.

L'invention concerne également un procédé tel que décrit ci-avant comportant une étape supplémentaire d'oxydation ou de réduction de la matière préalablement oxydée enzymatiquement.

Le procédé revendiqué permet notamment d'obtenir de manière simple, rapide et peu coûteuse des matières organiques, en particulier de nature saccharidique, oxydées avec une grande sélectivité puis éventuellement réduites ou ré-oxydées, présentant de nombreuses applications industrielles, y compris en tant qu'intermédiaires de synthèse, telles que la glucosone, la galactosone, les acides gluconique, 2-céto-gluconique ou isoascorbique, le fructose, le sorbitol, le mannitol,...

Par " matières organiques " au sens de la présente invention, on entend aussi bien les matières saccharidiques que les matières organiques non saccharidiques.

Ces dernières incluent les alcools et les acides organiques de nature non saccharidique tels que par exemple les alcools inférieurs dont le méthanol, les alcools gras, le glycérol, le cholestérol, les alcools polyvinyliques, les acides hydroxycarboxyliques dont l'acide malique, leurs dérivés respectifs et, de manière générale, tous les produits organiques autres que les saccharides, potentiellement aptes à être oxydés enzymatiquement avec production concomitante de peroxyde d'hydrogène.

Comme indiqué, le procédé objet de l'invention peut avantageusement être appliqué à des matières saccharidiques, cette notion étant aucunement limitative et comprenant l'ensemble des monosaccharides, disaccharides, trisaccharides, oligosaccharides et polysaccharides, linéaires, cycliques ou branchés, ainsi que les mélanges de ces produits tels que les hydrolysats d'amidon, d'inuline ou de cellulose.

Il est également applicable à des matières saccharidiques ayant subi, avant l'étape caractéristique d'oxydation enzymatique, au moins une étape de modification chimique, enzymatique et/ou physique, en particulier d'hydrolyse, d'oxydation ou d'hydrogénation et/ou au moins une étape de purification.

De préférence, la matière saccharidique est choisie dans le groupe comprenant les monosaccharides, les disaccharides, les dérivés oxydés ou hydrogénés des monosaccharides et disaccharides et les mélanges quelconques d'au moins deux quelconques de ces produits et ce, indépendamment du procédé ayant permis l'obtention de tels produits.

Les monosaccharides peuvent notamment consister en des pentoses ou des hexoses tels que le xylose, l'arabinose, le ribose, le glucose, le galactose, le mannose, le sorbose ou le fructose.

Les disaccharides peuvent notamment consister en du maltose, de l'isomaltose, du lactose, du lactulose, du cellobiose ou du saccharose.

Comme indiqué, le procédé selon l'invention est également applicable à des matières organiques constituées de monosaccharides ou disaccharides déjà modifiés, en particulier déjà oxydés ou hydrogénés, purifiés ou non.

Les monosaccharides et disaccharides oxydés soumis à l'étape caractéristique d'oxydation enzymatique selon l'invention peuvent notamment correspondre aux produits de l'oxydation, en un ou plusieurs endroits, des monosaccharides et disaccharides listés ci-avant et en particulier consister en l'un et/ou l'autre des acides gluconique, glucarique, 5-céto-gluconique, galactonique, galactarique, gulonique, maltobionique ou lactobionique, lesdits acides pouvant se présenter sous forme libre et/ou lactonisée et/ou de sels.

La forme lactonisée peut, à titre d'exemple, consister en une gluconolactone, une galactolactone ou une gulonolactone.

Les monosaccharides et disaccharides hydrogénés peuvent notamment correspondre aux produits de l'hydrogénation catalytique plus ou moins poussée, des monosaccharides et disaccharides précités.

Il est largement connu que des monosaccharides ou des disaccharides, éventuellement déjà oxydés ou hydrogénés, peuvent être oxydés enzymatiquement, en particulier par des oxydoréductases susceptibles d'utiliser l'oxygène comme accepteur d'hydrogène et donc aptes à générer du peroxyde d'hydrogène ou eau oxygénée (H₂O₂) dans le milieu réactionnel.

Il s'agit notamment d'enzymes du Groupe 1.1.3 tel que défini dans le document "ENZYME NOMENCLATURE", réédité périodiquement par l'International Union of Biochemistry and Molecular Biology. La nomenclature de telles enzymes est reprise par exemple en pages 55 à 60 de l'édition 1992 dudit document.

Il s'agit, entre autres, des enzymes présentant l'une au moins des activités Glucose oxydase, Hexose oxydase, Galactose oxydase ou Pyranose oxydase.

Ainsi, il est fait appel régulièrement, notamment depuis une vingtaine d'années, à l'usage de la Pyranose oxydase (également dénommée, entre autres, " Glucose 2-oxydase ", " Pyranose : oxygène 2-oxydoréductase " ou plus simplement " P2O ") pour transformer enzymatiquement des monosaccharides, éventuellement déjà oxydés, en leurs équivalents oxydés en position 2, et en particulier pour transformer le glucose en glucosone ou le galactose en galactosone. Ces produits sont des intermédiaires de synthèse de grand intérêt dans la voie d'obtention de produits comme le fructose, le sorbitol, le mannitol ou le tagatose qui, seuls ou en mélange, présentent de larges domaines d'application, en particulier dans les industries alimentaires, pharmaceutiques et chimiques.

Il est aussi fait largement appel, depuis plusieurs décennies, à l'usage de glucose oxydase (également dénommée, entre autres, " glucose oxyhydrase", " bêta-D-glucose : oxygène 1-oxydoréductase " ou plus simplement " GOD ") pour la préparation d'acide gluconique, sous forme libre, lactonisée et/ou de sels, à partir de glucose.

Cependant, l'un des inconvénients majeurs des enzymes précitées est de générer du peroxyde d'hydrogène et ce, de manière concomitante et équimoléculaire au produit oxydé souhaité. Or, il est reconnu que la présence de peroxyde d'hydrogène est globalement néfaste à l'activité d'une oxydoréductase telle que la pyranose ou la glucose oxydase et il est généralement recherché d'éliminer, en tout ou partie, le peroxyde d'hydrogène du milieu réactionnel au cours de la réaction d'oxydation enzymatique.

Différents moyens de nature enzymatique, chimique ou physique ont été proposés en vue d'éliminer ou de diminuer le peroxyde d'hydrogène ou pour le moins, les effets néfastes liés à la genèse et la présence de ce composé dans le milieu complexe que constitue le milieu d'oxydation enzymatique.

Le moyen le plus souvent décrit consiste en l'usage de catalase, sous forme libre ou immobilisée, en vue de décomposer enzymatiquement le peroxyde d'hydrogène. Cet usage est décrit dans de nombreux brevets tels que les brevets WO 81/03664 et WO 81/03666 publiés en 1981 au nom de STANDARD BRANDS, les brevets subséquents US 4,351,902 - US 4,423,149 - US 4,568,645 - US 4,569,910 - US 4,569,913 - US 4,569,915 et US 4,650,758 au nom de CETUS CORPORATION, WO 97/24454 au nom de GENENCOR INTERNATIONAL, INC et US 5,897,995 au nom de GIST-BROCADES B.V.

L'usage de catalase a également été décrit, entre autres, dans les récents articles scientifiques suivants :
- "Laboratory procedures for producing 2-keto-D-glucose, 2-keto-D-xylose and 5-keto-D-fructose from D-glucose, D-xylose and L-sorbose with immobilized pyranose oxidase of *Peniophora gigantea",* A. HUWIG et al, Journal of Biotechnology 32, 309-315 (1994),
- " A Convenient Enzymatic Procedure for the Production of Aldose-Free D-Tagatose", D. HALTRICH et al, ANNALS NEW YORK ACADEMY OF SCIENCES, 864, 295-299 (1998),
- " The CETUS Process revisited : a novel enzymatic alternative for the production of aldose-free D-fructose", C. LEITNER et al, Biocatalysis and Biotransformation, Vol. 00, 1-18 (1998).

D'autres voies déjà préconisées d'élimination/diminution du peroxyde d'hydrogène ou de ses effets néfastes dans un milieu réactionnel tel qu'envisagé ici, consistent en l'usage de moyens chimiques tels que :
- Les oxydes de manganèse qui seraient aptes à décomposer H₂O₂ selon l'article "The Immobilization of Glucose Oxidase to Manganese Oxide ", Z. DUVNJAK et al, BIOTECHNOLOGY AND BIOENGINEERING, VOL. XVIII, 737-739 (1976),
- Le sulfate de quinine ou l'urée qui seraient aptes à stabiliser la glucose oxydase en présence de H₂O₂ selon l'article "The Influence of Peroxide - Stabilizing Agents on Enzyme Deactivation by H₂O₂", Y.K. CHO et al, BIOTECHNOLOGY AND BIOENGINEERING, VOL. XIX, 157-158 (1977).
- Le charbon actif qui serait apte à inactiver H₂O₂ selon l'article "Enzyme Immobilization on Activated Carbon: Alleviation of Enzyme Deactivation by Hydrogen Peroxide", Y.K. CHO et al, BIOTECHNOLOGY AND BIOENGINEERING, VOL. XIX, 769-775 (1977),
- Les alcènes qui seraient aptes à " consommer" H₂O₂ et à former ainsi des glycols ou des oxydes d'alcoylène valorisables selon le brevet US 4,321,324 au nom de CETUS CORPORATION,
- Le platine qui, en présence de mycélium producteur de P2O et d'une solution diluée de glucose (2,5 %), serait apte à décomposer H202 selon le brevet WO 81/03666 précité,
- La stabilisation de la pyranose oxydase par amidination selon le brevet US 4,650,758 précité,
- La purification de la pyranose oxydase par élimination de l'activité parasite "pyranosone déhydratase", selon le brevet US 4,569,913 précité.

D'autres documents préconisent l'élimination (des effets néfastes) du peroxyde d'hydrogène par l'association d'un moyen physique (membrane semi perméable) et d'un moyen enzymatique (catalase) ou chimique (alcène) comme décrit dans les brevets WO 81/03664 et US 4,321,324 précités.

Cependant, force est de constater que l'état de la technique le plus récent, représenté par les exemples des brevets CETUS CORPORATION et articles scientifiques précités, n'envisage en pratique que la seule catalase comme moyen d'élimination principal du peroxyde d'hydrogène et de ses effets néfastes.

La catalase peut notamment être physiquement associée à l'oxydoréductase (pyranose oxydase, glucose oxydase notamment) par le biais de complexes enzymatiques naturellement présents dans différents microorganismes ou de pré-mélanges d'enzymes d'origines différentes, mais également au moyen d'une co-immobilisation sur un même support, ces variantes étant décrites, par exemple, dans les brevets US 4,569,910 - US 4,650,758 - US 4,351,902 et US 5,897,995 précités.

La catalase peut également être associée à de la sérum albumine bovine ("BSA") agissant comme agent stabilisant ou protecteur de l'oxydoréductase comme décrit dans les articles de HALTRICH et de LEITNER précités. Le mode précis d'action de la BSA serait mal connu mais, selon LEITNER, cette protéine pourrait notamment protéger la pyranose oxydase, mais aussi la catalase, des effets néfastes de la glucosone. D'autres agents protecteurs peuvent consister en caséine, méthionine, quinine ou en mannitol, sorbitol ou glycérol selon le brevet US 5,897,995.

En tout état de cause, il est connu que la catalase est également inactivée par le peroxyde d'hydrogène et qu'il convient, en pratique, de la mettre en oeuvre en grandes quantités, en tous cas en fort excès par rapport aux quantités d'oxydoréductases utilisées conjointement.

Les brevets WO 97/24454 et US 5,897,995, publiés très récemment, envisagent des ratios entre le nombre d'unités de catalase et le nombre d'unités d'oxydoréductase (glucose oxydase) utilisées dans le milieu réactionnel (ci-après ratios C/O), de l'ordre de 40-80 (WO 97/24454) voire au moins égaux à 140 (US 5,897,995).

En dernier lieu, les articles de HALTRICH et LEITNER précités préconisent un ratio C/O de 1000, associé à la mise en oeuvre de BSA à raison de 5 mg/ml de milieu réactionnel, l'oxydoréductase (pyranose oxydase) et la BSA pouvant, à l'inverse de la catalase utilisée, instable, être recyclées après avoir été extraites du milieu réactionnel par ultrafiltration.

Un autre enseignement de LEITNER concerne le fait que le platine déposé sur charbon actif ne peut, en fait, se substituer à la catalase puisque son utilisation résulte en une perte de plus de 95 % de l'activité P20 dans des conditions réactionnelles standard.

En suite de quoi, malgré le coût de mise en oeuvre de la catalase, il n'était pas envisagé sérieusement jusqu'alors, d'en remettre en cause l'intérêt et l'usage grandissants.

Il a désormais été mis au point, après de nombreuses recherches, un nouveau moyen de préparation efficace de matières organiques oxydées enzymatiquement comme par exemple la glucosone ou l'acide gluconique, moyen qui permet, si on le souhaite, d'obtenir lesdites matières en présence de quantités diminuées, voire nulles, de catalase et/ou d'agent protecteur tel que la BSA.

La Société Demanderesse a en particulier trouvé que, de matière surprenante et inattendue, le ruthénium et le palladium permettaient, à l'inverse notamment du platine, de se substituer avantageusement à la catalase et permettre à une oxydoréductase comme la pyranose ou la glucose oxydase, d'agir rapidement et ce, avec un rendement élevé et une excellente sélectivité.

De manière plus précise la présente invention a pour objet un procédé de transformation d'une matière organique comprenant une étape d'oxydation au cours de laquelle une matière organique est soumise à l'action oxydante d'un moyen enzymatique apte à générer du peroxyde d'hydrogène, ce procédé étant caractérisé par le fait que ladite étape d'oxydation est menée, en tout ou partie, en présence d'un métal choisi parmi le ruthénium, le palladium et leurs mélanges.

De manière avantageuse, ledit métal est présent à raison de 0,001 à 1 %, de préférence de 0,005 à 0,2 %, ces pourcentages étant exprimés en poids sec total de ruthénium et palladium par rapport au poids sec total du milieu réactionnel.

Comme indiqué, il n'est pas obligatoire que ledit métal soit présent dans le milieu réactionnel de manière parfaitement simultanée au moyen d'oxydation enzymatique, l'un pouvant être introduit dans le milieu réactionnel et/ou retiré du milieu réactionnel avant l'autre. Le ruthénium et/ou le palladium peuvent être introduits sous toutes formes appropriées et en particulier sous forme immobilisée sur support, obtenue par toute méthode connue, notamment d'imprégnation ou d'échange ionique. Le support peut être constitué par exemple, de charbon actif, de tourbe, de zéolithe, de dioxyde de titane, ou d'un polymère synthétique de type fibre de carbone. Un intérêt indéniable du procédé ici revendiqué est de permettre le recyclage dudit métal, ce qui est généralement impossible avec une catalase ordinaire.

Les matières organiques, en particulier de nature saccharidique, pouvant servir de substrat à l'un, au moins, des moyens d'oxydation enzymatique ici envisagés, ont été décrites ci-avant et concernent notamment les monosaccharides, les disaccharides et leurs dérivés respectifs, par exemple déjà oxydés.

Par " moyen d'oxydation enzymatique " dans le cadre de la présente invention, on entend en particulier les oxydoréductases du groupe 1.1.3 au sens du document " ENZYME NOMENCLATURE " précité et les mélanges d'au moins deux quelconques de ces enzymes, étant précisé que lesdites enzymes peuvent être mises en oeuvre, sous forme libre ou immobilisée sur support, en tant que telles, sous forme partiellement ou totalement purifiée et/ou au moyen d'organismes synthétisant lesdites enzymes.

A titre d'exemples, le moyen d'oxydation enzymatique peut être choisi parmi les enzymes ou mélanges d'enzymes présentant l'une au moins des activités Glucose oxydase, Hexose oxydase, Galactose oxydase, Pyranose oxydase, L-Sorbose oxydase, Cellobiose oxydase, L-Gulonolactone oxydase, L-Galactonolactone oxydase, Alcool oxydase, Alcool secondaire oxydase ou (S)-2-Hydroxyacide oxydase ainsi que leurs équivalents respectifs, actuels ou futurs.

De manière avantageuse, le moyen d'oxydation enzymatique présente l'une au moins des activités Glucose oxydase, Hexose oxydase, Galactose oxydase ou Pyranose oxydase.

Il peut s'agir notamment d'une préparation à base d'enzyme(s), partiellement ou totalement purifiée(s), sous forme libre ou immobilisée sur support, présentant une activité Pyranose oxydase et/ou Glucose oxydase ou d'organisme(s), sous forme libre ou immobilisée sur support, synthétisant ce type d'enzyme(s).

De tels organismes, préparations enzymatiques et enzymes, lesquels peuvent avantageusement être recyclés, sont décrits dans les brevets et articles scientifiques précités, lesquels font partie intégrante de la présente description. Ils peuvent, à titre d'exemples, être issus de (consister en) l'un ou l'autre des organismes suivants :
- *Polyporus obtusus, Trametes multicolor, Coriolus versicolor, Lenzites betulinus, Oudemansiella mucida, Aspergillus flavus,* aptes à synthétiser, entre autres, de la Pyranose oxydase,
- *Aspergillus niger, Aspergillus oryzae* aptes à synthétiser, entre autres, de la Glucose oxydase,
ainsi que tous leurs équivalents taxonomiques et/ou fonctionnels, actuels ou futurs, d'origine naturelle ou non, résultant notamment de traitements de mutations ou de manipulations génétiques.

De préférence, le moyen d'oxydation enzymatique présente une activité Pyranose oxydase.

Selon une variante du procédé selon l'invention, l'étape d'oxydation enzymatique est menée en présence d'un ratio d'activité catalase/oxydoréductase (ratio C/O) tel que précisé ci-avant, inférieur à 1000 et, en particulier :
- inférieur à 500, de préférence inférieur à 200, quand l'oxydoréductase consiste en Pyranose oxydase, ou
- inférieur à 40 quand l'oxydoréductase consiste en Glucose oxydase.

Il convient de rappeler ici qu'une préparation d'oxydoréductase peut, malgré les efforts continus visant à la purification de l'enzyme, présenter simultanément une activité catalase plus ou moins importante et qui lui est plus ou moins intimement associée physiquement. Ceci, du fait notamment d'une purification encore imparfaite et/ou d'un recyclage à partir d'un milieu ayant également contenu de la catalase. La mise en oeuvre de ladite oxydoréductase peut donc impliquer, notamment du fait de son recyclage, la mise en oeuvre concomitante d'une activité catalase " endogène " qu'il convient de distinguer d'une activité catalase " exogène ", i.e apportée de manière prédéterminée, indépendamment de l'activité oxydoréductase, simultanément ou non à celle-ci.

Selon une variante du procédé selon l'invention, l'étape d'oxydation enzymatique est menée en absence de tout apport d'activité catalase exogène.

Selon une autre variante, le moyen d'oxydation enzymatique et le métal (ruthénium et/ou palladium) sont immobilisés sur un même support.

La concentration de la matière organique utilisée comme substrat du moyen d'oxydation enzymatique n'est soumise à aucune contrainte particulière en regard de l'art antérieur. La matière organique peut notamment être apportée sous la forme d'une solution présentant une matière sèche (" MS ") comprise entre 2 et 70 %, en particulier entre 4 et 60 %. Il peut s'agir, par exemple, d'une solution de glucose présentant une MS supérieure à 5 %, notamment comprise entre 6 et 55 %, utilisable comme substrat de la Pyranose oxydase ou de la Glucose oxydase. En pratique, la réaction d'oxydation enzymatique est généralement menée à une température comprise entre 15 et 60°C, par exemple comprise entre 20 et 35°C quand le moyen d'oxydation enzymatique consiste en Pyranose ou Glucose oxydase.

Les autres paramètres réactionnels sont ceux généralement rencontrés dans la littérature, y compris en ce qui concerne l'aération, l'agitation et le pH du milieu réactionnel.

La Société Demanderesse a cependant trouvé que dans le cas de la Pyranose oxydase, le pH du milieu réactionnel se situait avantageusement au dessus de 5,5 environ et notamment entre 5,6 et 6,5 environ.

Comme indiqué, le procédé selon l'invention permet de manière remarquable l'obtention d'une matière oxydée comme la glucosone avec des rendement et sélectivité très élevés à partir de glucose et ce, dans des délais très courts. Lorsque le moyen d'oxydation présente l'activité Pyranose oxydase, le procédé selon l'invention peut donc avantageusement être caractérisé par le fait que l'étape d'oxydation enzymatique présente une durée inférieure à 6 heures, de préférence inférieure à 5 heures.

En suite de quoi, on dispose désormais d'un nouveau moyen, simple, efficace, peu coûteux et très sélectif d'obtention de matières organiques oxydées.

Ce moyen est particulièrement adapté à la préparation d'une composition saccharidique contenant au moins un monosaccharide ou un disaccharide, de préférence choisi dans le groupe comprenant le glucose, le galactose, le mannose, le xylose, le sorbose, le maltose, le lactose et les mélanges quelconques d'au moins deux quelconques de ces produits, oxydé en au moins un endroit et éventuellement lactonisé, en particulier à la préparation d'une composition à base d'au moins un produit choisi dans le groupe comprenant la glucosone, la galactosone, la xylosone, la glucosyl-glucosone, le 2,5-dicéto-fructose, l'acide gluconique, l'acide galactonique, l'acide 2-céto-gluconique, l'acide 2-céto glucarique, l'acide 2,5 di-céto-gluconique, l'acide isoascorbique, lesdits acides se présentant sous forme libre et/ou lactonisée et/ou de sels, et les mélanges quelconques d'au moins deux quelconques desdits produits.

Du fait notamment de leur pureté, les matières organiques oxydées obtenues conformément à l'invention peuvent avantageusement être soumises, si on le souhaite, à une ou plusieurs étapes subséquentes de modification, notamment de nature chimique.

La présente invention concerne en particulier un procédé tel que décrit précédemment caractérisé en ce qu'il comprend, subséquemment à l'étape d'oxydation enzymatique en présence de ruthénium et/ou palladium, au moins une étape supplémentaire de réduction ou d'oxydation de la matière organique oxydée enzymatiquement, éventuellement purifiée.

L'étape de réduction peut se faire par voie enzymatique comme décrit dans les articles de HALTRICH et LEITNER précités. Avantageusement, il s'agit d'une étape d'hydrogénation catalytique comme décrit dans les brevets WO 81/03666 - US 4,321,324 et US 4,423,149 précités.

A titre d'exemple, la préparation de glucosone obtenue à partir de Glucose en présence de Pyranose oxydase et de ruthénium et/ou palladium, peut, du fait notamment de sa faible teneur en acide formique ou en autres espèces agissant comme poisons de catalyseurs, être avantageusement soumise à une étape supplémentaire d'oxydation ou d'hydrogénation catalytique.

Un autre intérêt économique indéniable du procédé selon l'invention est d'ailleurs de permettre le recyclage du ruthénium et/ou du palladium utilisé lors de l'étape d'oxydation enzymatique, aux fins de sa mise en oeuvre lors d'une étape subséquente d'hydrogénation catalytique.

Selon une variante, ce procédé est donc caractérisé en ce qu'il comprend une étape supplémentaire d'hydrogénation catalytique, continue ou discontinue, celle-ci étant de préférence également menée en présence de ruthénium et/ou de palladium, recyclé ou non à partir de l'étape antérieure d'oxydation enzymatique.

Ce procédé peut donc être avantageusement utilisé pour la préparation d'une composition saccharidique contenant au moins un produit choisi dans le groupe comprenant le fructose, le sorbitol, le mannitol, le tagatose, hydrogéné ou non, le glucosyl-sorbitol, le glucosyl-mannitol et les mélanges quelconques d'au moins deux quelconques de ces produits.

Selon une autre variante du procédé selon l'invention, l'étape d'oxydation enzymatique est menée de manière continue. Ainsi, la matière organique devant être soumise à ladite étape est introduite, en continu, dans le milieu réactionnel et la matière organique oxydée ayant subi ladite étape, par exemple la glucosone, est soutirée, également en continu, dudit milieu réactionnel.

Dans ce cas, le ruthénium et/ou palladium (sur support) peut avantageusement être utilisé sous forme de granulés, lesquels sont placés dans une enceinte perforée et généralement fixe, immergée dans le milieu réactionnel. Les granulés métalliques peuvent ainsi rester en contact continu avec le milieu réactionnel.

Par ailleurs, la matière organique oxydée peut, selon une autre variante, avantageusement subir, de manière également continue ou non, au moins une étape subséquente de filtration, par exemple de microfiltration et/ou d'ultrafiltration en vue notamment de pouvoir recycler, de manière continue ou non, en tout ou partie, le moyen d'oxydation enzymatique et/ou le métal (ruthénium et/ou palladium) préalablement utilisé. La matière organique oxydée ainsi purifiée, par exemple la glucosone, peut alors subir, comme déjà indiqué, une étape subséquente d'hydrogénation, notamment continue.

La présente invention va être décrite de façon encore plus détaillée à l'aide des exemples qui suivent et qui ne sont aucunement limitatifs.

### EXEMPLE 1

Dans un réacteur de 2 l, on introduit 1 l d'une solution de glucose à 10 %, 1 ml d'antimousse (PEG), 5 g de sérum albumine bovine (BSA) et 2000 unités de Pyranose oxydase (P20) apportées sous la forme d'une préparation enzymatique liquide issue de *Trametes multicolor* et présentant une activité catalase endogène telle que son ratio d'activité catalase / oxydoréductase (ratio C/O) est de 50/l environ.

L'activité de la catalase est mesurée selon la méthode UV décrite en point 3.9.1 dans "Methods of Enzymatic Analysis " de BERGMEYER et al - 3^{ème} édition, 1983 Vol III, " Enzymes 1 : Oxidoreductases, Transferases ".

L'activité de la Pyranose oxydase est mesurée selon l'article de LEITNER précité.

Le début de la réaction (TO) est défini comme correspondant au moment ou glucose et P20 sont mis en présence l'un de l'autre.

Le milieu réactionnel est maintenu sous agitation (500 tours/minute), aération (1 1 d'air / 1 milieu / minute) et température (30°C) constantes. Le pH du milieu est régulé à une valeur de 5,8 à l'aide de bicarbonate de potassium 1 M.

Au cours de cet essai (ESSAI T1), on mesure régulièrement la teneur du milieu réactionnel en produit oxydé souhaité, en l'occurrence en glucosone (ci-après " GL02 ") mais aussi, éventuellement, en glucose résiduel (" GLU ") ainsi qu'en produits oxydés non souhaités tels que les acides arabinonique (sous forme libre et lactonisée - collectivement "ARA"), formique (" FOR ") ou acétique (" ACE ").

On effectue les ESSAIS T2 à T4 ainsi que A à E dans les mêmes conditions générales que celles utilisées pour l'ESSAI T1 si ce n'est que l'on procède, à TO ou aux environs de TO, à l'introduction supplémentaire dans le milieu réactionnel de, respectivement :
- ESSAI T2 : 2 g de charbon actif" SX PLUS " de NORIT,
- ESSAI T3 : 8 x 10⁴ unités de catalase exogène " SIGMA C 30 ",
- ESSAI T4 : 3 x 10⁶ unités de catalase exogène " SIGMA C 30 ",
- ESSAI A : 1,16 g de platine sur charbon actif apportant environ 0,05 % de métal (Pt), exprimé en poids sec de métal/poids sec total du milieu réactionnel,
- ESSAI B : 2 g de nickel de Raney apportant 2 % environ de métal (Ni),
- ESSAI C : 2 g de cuivre de Raney apportant 2 % environ de métal (Cu),
- ESSAI D : 2 g de ruthénium sur charbon actif apportant 0,05 % environ de métal (Ru),
- ESSAI E : 1,37 g de palladium sur charbon actif apportant 0,05 % environ de métal (Pd).

Pour chacun des ESSAIS T1 à T4 et A à E, on obtient à TX, i.e X heures après TO, les résultats ci-après en termes de concentration du milieu réactionnel en glucosone (" GLO2 ") et, éventuellement, en autres composés, les pourcentages étant exprimés en poids sec du produit recherché sur le poids sec total du milieu réactionnel.

| ESSAI | TX (HEURES) | GLO2 % | GLU % | ARA % | FOR % | ACE % |
|---|---|---|---|---|---|---|
| T1 | 4 | 38,3 | 60,9 | NR | NR | NR |
| T2 | 4 | 86,8 | 12,2 | NR | NR | NR |
| T3 | 6 | 74 | 25 | NR | NR | NR |
| T4 | 4 | 54,5 | 45,1 | abs | 0,08 | NR |
| A | 5 | 48,0 | 48,5 | NR | NR | NR |
| B | 3 | 78,8 | 14,2 | 4,2 | 1,5 | 1,3 |
| C | 3,5 | 22,5 | 75 | NR | NR | NR |
| D | 4 | 98,7 | abs | 1,0 | 0,2 | 0,06 |
| E | 4,5 | 95,0 | abs | 4,1 | 0,8 | < 0,02 |
| NR = produit non recherché lors de l'analyse | | | | | | |
| abs = produit non détecté lors de l'analyse | | | | | | |

Ces résultats montrent globalement que des métaux comme le platine mais également le nickel et le cuivre, ne peuvent pas être mis en oeuvre efficacement au sein des milieux complexes et particuliers que constituent les milieux d'oxydation enzymatique ici envisagés.

Des analyses supplémentaires ont par ailleurs montré :
a) qu'en présence de platine, l'activité Pyranose oxydase était, après 5 heures de réaction, inhibée à raison de 90 % environ et que la poursuite de la réaction ne donnait lieu qu'à une faible augmentation de la concentration en Glucosone, une partie significative (≈ 10 %) du Glucose résiduel se transformant par ailleurs en coproduits non souhaités,
b) qu'en présence de cuivre, l'activité Pyranose oxydase était totalement inhibée au bout de 3,5 heures de réaction, un ajoût supplémentaire de 2000 unités de Pyranose oxydase étant lui-même totalement rendu inefficace en 15 minutes.
c) qu'en présence de nickel, plus de 40 % de l'activité Pyranose oxydase était inhibée en 3 heures de réaction avec en outre une solubilisation très significative (≈ 30 %) du métal dans le milieu.

A l'inverse, ces résultats montrent que de manière très surprenante, le ruthénium et le palladium peuvent avantageusement être associés à une oxydoréductase comme la Pyranose oxydase en vue d'obtenir de la glucosone dans les délais très courts, i.e de l'ordre de 4-5 heures et ce, avec une conversion complète du glucose et une sélectivité très élevée.

En absence de tels métaux et y compris avec un ratio catalase (endogène + exogène) / oxydoréductase particulièrement élevé, à savoir supérieur à 1,5 10³ comme dans le cas de l'ESSAI T4, on n'arrive pas à obtenir, en 4 heures de réaction, un taux de conversion du glucose qui atteigne 60 %.

Le charbon actif permet d'améliorer ce taux de conversion et d'atteindre, en 4 heures, une valeur proche de 87 % (86,8 % - cf ESSAI 2). Cependant, la Société Demanderesse a constaté que si on laisse la réaction se poursuivre ainsi, i.e en présence de charbon actif mais en l'absence de ruthénium et/ou palladium, la réaction ralentit significativement avec génèse de quantités importantes de coproduits non souhaités. Ainsi, après 5 heures de réaction, on note une production de glucosone encore inférieure à 90 % (89,8 %) avec un taux résiduel de glucose très significatif (3,5 %) et un taux de coproduits non souhaités relativement important (6,3 %).

Un essai complémentaire, identique à l'essai D si ce n'est que l'on a introduit 0,125 % de ruthénium (au lieu de 0,05 %), a confirmé la possibilité d'obtenir, en 4 heures de réaction, un taux de conversion du glucose qui soit au moins égal à 90 % et ce, avec une sélectivité également remarquable.

### EXEMPLE 2

Dans cet exemple, on a étudié l'intérêt de la mise en oeuvre de 0,005 % de ruthénium en association avec une préparation de Glucose oxydase (1000 unités / 1 milieu) comportant une activité catalase endogène (rapport d'activité catalase / oxydoréductase de 7/1) et ce, en vue de la transformation du glucose (solution à 150 g/l) en acide gluconique.

Après 5 heures de réaction à 33°C et à pH 5,5 (régulation par NaOH à 50 %), il a été observé qu'en présence de ruthénium, le taux de conversion du glucose en acide gluconique (≈ 34 %) était au moins 3 fois supérieur à celui obtenu en absence d'un tel métal (≈ 10 %).

## Revendications

1. Procédé de transformation d'une matière organique comprenant une étape d'oxydation au cours de laquelle une matière organique est soumise à l'action oxydante d'un moyen enzymatique apte à générer du peroxyde d'hydrogène, ce procédé étant caractérisé par le fait que ladite étape d'oxydation est menée, en tout ou partie, en présence d'un métal choisi parmi le ruthénium, le palladium et leurs mélanges.

2. Procédé selon la revendication 1 caractérisé par le fait que le métal est présent à raison de 0,001 à 1 %, de préférence de 0,005 à 0,2 %, ces pourcentages étant exprimés en poids sec total de ruthénium et palladium par rapport au poids sec total du milieu réactionnel.

3. Procédé selon l'une des revendications 1 ou 2 caractérisé par le fait que la matière organique soumise à l'étape d'oxydation enzymatique est une matière saccharidique, de préférence choisie dans le groupe comprenant les monosaccharides, les disaccharides, les dérivés oxydés ou hydrogénés des monosaccharides et disaccharides et les mélanges quelconques d'au moins deux quelconques de ces produits.

4. Procédé selon l'une quelconque des revendications 1 à 3 caractérisé par le fait que le moyen d'oxydation enzymatique est choisi parmi les oxydoréductases du groupe 1.1.3 et les mélanges quelconques d'au moins deux quelconques de ces enzymes, lesdites enzymes étant mises en oeuvre, sous forme libre ou immobilisée sur support, en tant que telles, sous forme partiellement ou totalement purifiée et/ou au moyen d'organismes synthétisant lesdites enzymes.

5. Procédé selon la revendication 4 caractérisé par le fait que le moyen d'oxydation enzymatique est choisi parmi les enzymes ou mélanges d'enzymes présentant l'une au moins des activités Glucose oxydase, Hexose oxydase, Galactose oxydase ou Pyranose oxydase, de préférence présentant l'une au moins des activités Glucose oxydase ou Pyranose oxydase, et plus préférentiellement encore l'activité Pyranose oxydase.

6. Procédé selon l'une quelconque des revendications 1 à 5 caractérisé par le fait que l'étape d'oxydation enzymatique est menée en présence d'un ratio d'activité catalase / oxydoréductase inférieur à 1000 et, en particulier :
- inférieur à 500, de préférence inférieur à 200, quand l'oxydoréductase consiste en Pyranose oxydase, ou
- inférieur à 40 quand l'oxydoréductase consiste en Glucose oxydase.

7. Procédé selon l'une quelconque des revendications 1 à 6 caractérisé par le fait qu'il comprend, subséquemment à l'étape d'oxydation enzymatique, au moins une étape supplémentaire de réduction ou d'oxydation de la matière organique oxydée enzymatiquement, éventuellement purifiée.

8. Procédé selon la revendication 7 caractérisé par le fait que l'étape supplémentaire consiste en une étape d'hydrogénation catalytique, continue ou discontinue, celle-ci étant de préférence également menée en présence de ruthénium et/ou de palladium, recyclé ou non à partir de l'étape antérieure d'oxydation enzymatique.

9. Utilisation du procédé selon l'une des revendications 1 à 7 pour la préparation d'une composition saccharidique contenant au moins un monosaccharide ou un disaccharide, de préférence choisi dans le groupe comprenant le glucose, le galactose, le mannose, le xylose, le sorbose, le maltose, le lactose et les mélanges quelconques d'au moins deux quelconques de ces produits, oxydé en au moins un endroit et éventuellement lactonisé, en particulier pour la préparation d'une composition à base d'au moins un produit choisi dans le groupe comprenant la glucosone, la galactosone, la xylosone, la glucosyl-glucosone, le 2,5-dicéto-fructose, l'acide gluconique, l'acide galactonique, l'acide 2-céto-gluconique, l'acide 2-céto glucarique, l'acide 2,5 di-céto-gluconique, l'acide isoascorbique, lesdits acides se présentant sous forme libre et/ou lactonisée et/ou de sels, et les mélanges quelconques d'au moins deux quelconques desdits produits.

10. Utilisation du procédé selon l'une des revendications 7 ou 8 pour la préparation d'une composition saccharidique contenant au moins un produit choisi dans le groupe comprenant le fructose, le sorbitol, le mannitol, le tagatose, hydrogéné ou non, le glucosyl-sorbitol, le glucosyl-mannitol et les mélanges quelconques d'au moins deux quelconques de ces produits.
